# EUROPEAN PATENT SPECIFICATION

(11) **EP 2 203 146 B1**
(45) Date of publication and mention of the grant of the patent: **21.08.2013**
(21) Application number: 08843020.2
(22) Date of filing: 20.10.2008
(51) Int. Cl.: A61K 8/03, A61Q 19/00

(54) **MULTI-FUNCTIONAL, MULTI-PHASE SKIN CARE PRODUCT**
MULTIFUNKTIONELLES MEHRPHASEN-HAUTPFLEGEPRODUKT
PRODUIT DE SOINS POUR LA PEAU, MULTI-PHASES, MULTIFONCTIONNEL

(30) Priority: 25.10.2007 US 417
(43) Date of publication of application: 07.07.2010
(73) Proprietor: The Procter & Gamble Company, Cincinnati, OH 45202 (US)
(72) Inventor: TANNER, Paul, Robert, Lebanon Ohio 45036 (US); ELSBROCK, Robert, John, Cincinnati Ohio 45243 (US)
(74) Representative: Engisch, Gautier
(86) International application number: PCT/IB2008/054319
(87) International publication number: WO 2009/053898

(56) References cited:
- EP-A- 1 327 439
- WO-A-02/100358
- WO-A-2006/102113
- DE-A1- 3 009 763
- GB-A- 2 259 015
- US-A1- 2004 223 991

## Description

### FIELD OF THE INVENTION

The present invention relates to a skin care product comprising a non-solid, multi-phase skin care composition comprising a translucent base phase, a first opaque phase, and a second opaque phase. The three phases form a stable, visually distinct pattern within a container that is at least partially translucent.

### BACKGROUND OF THE INVENTION

Skin care compositions are well known and widely used. Skin care compositions have long been employed and formulated to address specific functionalities such as cleansing, moisturization, regulating the signs of anti-aging (e.g., reducing the appearance of wrinkles and coarse deep lines, fine lines, crevices, bumps, and large pores), hiding imperfections, or to reduce the oiliness/shine associated with sebum. Personal care compositions have also been used to alter the color and appearance of skin. Many skin care compositions attempt to combine two functionalities in order to provide a more holistic product that can simplify the skin care and/or make-up regimen. For example, tinted moisturizers proving the benefits of moisturization and skin tone adjustment are well known. However, providing a multiple function skin care composition that provides skin tone adjustment can present difficulties with formulation and consumer acceptance.

Multiple function skin care compositions providing skin tone adjustment suffer a common deficiency. The colorants used to provide even skin tone yield a relatively homogenously tinted composition. This results in a multiple function skin care composition that appears no different from a make-up concealer (*i.e*., a single functional product). The composition itself provides no indication to the consumer that it is multiple function. This is a particularly difficult problem for manufacturers of multiple function skin care compositions. A multiple function skin care composition naturally includes additional functional actives which are not included in a single function skin care composition. These additional functional actives often are the most expensive raw ingredient within the skin care composition, which must be priced accordingly. Unfortunately, the additional functional actives are not readily appreciated by a consumer. Because of the colorant load necessary to provide skin tone adjustment, the multiple function skin care composition looks similar on a store shelf to a lower priced concealer. A consumer is unlikely to pay a premium for a multiple function skin care composition that is visually indistinguishable from a lower price single function concealer.

Another problem associated with multiple function skin care compositions is that the composition may provide more than two functionalities. As a result, the skin care composition must provide some cue or indication to consumers that the composition is multi-functional. Even if the homogenous tinting problem associated with colorants is solved, manufacturers are still left with a skin care composition that does not clearly communicate the multi-functionality versus a single or dual functional product. Again, the problem is exacerbated on the store shelf when consumers are hesitant to pay a premium for a multi-functional product that appears no different from a single or dual functional product.

One common means for communicating a multiple function product is through the use of a dual-chamber package. These packages have a first composition in one chamber and a second composition in another chamber. The package includes a means for dispensing from both containers simultaneously. These packages can be designed to highlight the distinct chambers. However, the dual-changed packages and the means for simultaneous dispensing may add considerable cost to the product.

Another method for communicating a multiple function product is through the use of a multi-phase composition. Most existing compositions exhibiting more than one phase are directed to dual-phase compositions. However, little direction is provided in the existing compositions or art for creating a stable multi-fuctional skin care composition having visually distinct phases. Stability problems are not trivial in multi-phase compositions. Unlike a homogenous (*i.e.*, not multi-phase) composition where an ingredient is distributed relatively evenly throughout the entire composition, multi-phase composition are often formulated such that the ingredient is in a single phase. This results in the ingredient representing a greater weight percentage in the single phase than in the composition as a whole. Ingredients such opacifying particulate materials, colorants, and sunscreens can cause stability problems when representing a substantial portion of a phase.

Furthermore, formulation of a multi-phase skin care composition is further complicated when one of the phases provides skin tone adjustment through the use of a high load of colorant or opacifying particulate material. For example, a multi-phase product that separates the colorant into a single phase is prone to cause streaking when applied to the skin because the colorant is not evenly distributed throughout the bulk of the composition.

### SUMMARY OF THE INVENTION

The present invention relates, in one embodiment, to a skin care product comprising a non-solid, multi-phase multi-function skin care composition comprising a translucent base phase, a first opaque phase, and a second opaque phase. The three phases form a stable, visually distinct pattern within a container that is at least partially translucent. The first opaque phase may have a lightness of greater than about 80 and a chroma of less than about 5. The second opaque phase may have a hue from about 35 to about 70. Lightness (L), chroma (C), and hue (H) are well known variables within the Commission Internationale de l'Eclairage L*a*b* color space (hereinafter "CIELab") and may be measured by the Colorimetery Method described hereafter.

The present invention further relates, in another embodiment, to a skin care product comprising a non-solid, multi-phase multi-function skin care composition comprising a translucent base phase comprising a first chronic skin care active, a first opaque phase comprising a skin conditioning agent, and a second opaque phase comprising at least about 1% of one or more opacifying particulate materials. The first opaque phase may have a lightness of greater than about 80 and a chroma of less than about 5. The second opaque phase may have a hue from about 35 to about 70. The three phases form a stable, visually distinct pattern within a container that is at least partially translucent.

The present invention further relates, in another embodiment, to a skin care product comprising a non-solid multi-phase multi-function skin care composition comprising a translucent base phase comprising a first chronic skin care active; a first opaque phase comprising a skin conditioning agent; and a second opaque phase comprising at least about 1% of one or more opacifying particulate materials. The first opaque phase may have a lightness of greater than about 80 and a chroma of less than about 5. The second opaque phase may have a hue from about 35 to about 70. The three phases form a stable, visually distinct pattern within a container that is at least partially translucent. The skin care product further comprises a plurality of informational indicia including a first informational indicia communicating a benefit of the translucent base phase, a second informational indicia communicating a benefit of the first opaque phase, and a third informational indicia communicating a benefit of the second opaque phase.

The present invention further relates, in another embodiment, to a skin care product comprising an array of skin care products comprising a first skin care product and a second skin care product. The first skin care product comprises a first non-solid multi-phase multi-function skin care composition comprises a translucent base phase, a first opaque phase which may have a lightness of greater than about 80 and a chroma of less than about 5, and a second opaque phase which may have a hue from about 35 to about 70. The three phases form a stable, visually distinct pattern within a first container that is at least partially translucent. The second skin care product comprises a second non-solid multi-phase multi-function skin care composition comprises a translucent base phase, a first opaque phase which may have a lightness of greater than about 80 and a chroma of less than about 5, and a second opaque phase which may have a hue from about 35 to about 70. The three phases form a stable, visually distinct pattern within a second container that is at least partially translucent. The first skin care product and second skin care product differ in that the second opaque phase of the first multi-phase skin care composition and the second opaque phase of the second multi-phase skin care composition exhibit a lightness difference (ΔL) of at least 4.

Additional attributes applicable to one or more embodiments of the present invention are discussed below.

### DETAILED DESCRIPTION OF THE INVENTION

As used herein, the following terms shall have the meaning specified thereafter. It should be recognized that capitalization of the listed terms is predicated on grammar and may not be continued throughout the remaining of the text.

"Phase" refers to a domain or region of a composition having one average composition, as distinct from another region or domain having a different average composition, wherein the domains are visible to the naked eye.

"Multi-phase" refers to at least three phases that occupy separate spaces with a container wherein each phase is in contact with at least one other phase (e.g., the phases are not stored in separate partitions in a container).

"Multi-function" (or "multi-functional") refers to a skin care composition that simultaneously performs or provides at least three distinct functions or benefits. Each distinct function or benefit may be attributable to a phase or an ingredient within the phase.

"Stable" means that there is substantially no mixing of the phases, observable to the naked eye, prior to dispensing of the composition. Commercials products should remain stable for at least 180 days to account for the time of manufacture, shipping, storage, display, purchase, and use.

"Non-solid" means that a material is (i) generally flowable to some degree at ambient conditions or (ii) exhibits a viscosity of equal to or greater than about 3,000 cps to about 1,000,000 cps, as measured by the Viscosity Method described hereafter.

"Visually distinct" means that the phases can be separately seen by the human eye as distinctly separate regions (*i.e.*, not emulsions or dispersions of particles).

"Translucent" means that a material (*e.g*., phase or container) is sufficiently clear such that the pattern of one or more opaque phases may be discerned.

"Informational Indicia" means any graphic, symbol, icon, word, phrase, or other marking that communicates a function or benefit to a user.

"Opacifying particulate material" refers to a non-fluid material having a refractive index of greater than 1.9. Refractive index can be determined by conventional methods.

"Ambient conditions" means at 25°C, under about one atmosphere of pressure, and at about 50% relative humidity

All ratios are weight ratios, unless specifically stated otherwise. All ranges are inclusive and combinable; therefore, every range given throughout this specification will include every narrower range that falls within such broader range as if such narrower ranges were all expressly written herein. The number of significant digits conveys neither a limitation on the indicated amounts nor on the accuracy of the measurements. All measurements are understood to be made at ambient conditions. All such weights as they pertain to listed ingredients are based on the active level and do not include carriers or by-products that may be included in commercially available materials, unless otherwise specified.

Multi-phase Skin Care Composition The multi-phase skin care composition of the present invention comprises at least three distinct phases including a translucent base phase, a first opaque phase, and a second opaque phase. The percentages of the individual phases may be adjusted as needed to produce a visually distinct pattern. The multi-phase skin care composition may comprise about 30 to about 90% of the translucent base phase, about 5 to about 30% of the first opaque phase, and about 5 to about 50% of the second opaque phase. The multi-phase skin care composition, in other embodiments, may comprise from about 40% to about 85% of the translucent base phase or from about 55% to about 75% of the translucent base phase. The multi-phase skin care composition, in other embodiments, may comprise, from about 5% to about 20% of the first opaque phase or from about 5% to about 15% of the first opaque phase. The multi-phase skin care composition, in other embodiments, may comprise, from about 10% to about 40% of the second opaque phase or from about 20% to about 30% of the second opaque phase. The skin care composition may further comprise one or more supplemental phases that form a visually distinct pattern,

The present invention relates, in one embodiment, to a skin care product comprising a non-solid, multi-phase skin care composition comprising a translucent base phase, a first opaque phase, and a second opaque phase. The three phases form a stable, visually distinct pattern within a container that is at least partially translucent. Suitable patterns include but are not limited to the following examples: striped, marbled, rectilinear, interrupted striped, check, mottled, veined, clustered, speckled, geometric, spotted, ribbons, helical, swirl, arrayed, variegated, textured, grooved, ridged, waved, sinusoidal, spiral, twisted, curved, cycle, streaks, striated, contoured, anisotropic, laced, weave or woven, basket weave, spotted, and tessellated. In one embodiment, the pattern includes the repeating pattern such as a repeating sine wave or repeating bead.

The non-solid, multi-phase skin care composition may be multi-functional. In embodiments where each phase corresponds to a different functionality, each phase of the skin care composition may be dispensed simultaneously to provide the multiple functions. The visually distinct pattern may be selected such that the multiple phases are dispensed simultaneously. In contrast, a multi-functional skin care composition may not be feasible when the visually distinct pattern is random (*e.g*., no uniformity in phase delivery) or striated (*e.g.*, a first layer is dispensed until depletion then a second layer is dispensed).

Translucent Base Phase - The translucent base phase may be in any form that exhibits translucency. Specifically, the base phase may be, for example, an emulsion or a gel. In certain embodiments, the base phase is a gel phase such as an aqueous gel, an anhydrous hydrocarbon gel, or an anhydrous silicone gel. Gels typically comprise a gelling agent and a solvent. For aqueous gels, water is a standard solvent; however, water may be substituted with or supplemented with other aqueous solvents such as polyols (*e.g*., glycerine, butylene glycol, etc.). Suitable gelling agents for aqueous gels include xanthan gum, polysaccharides, carbomers, and polymers and copolymers of acrylic acid, acylamide, acryloyldimethyl taurate and their derivatives. Commercially available gelling agents include Sepigel 305, Simulgel INS-100, Simulgel SMS 88, and Simulgel EG; all available from Seppic, Inc.

The translucent base layer may comprise an anhydrous hydrocarbon gel. Hydrocarbon solvents can be volatile or non-volatile. Suitable hydrocarbon solvents include saturated, unsaturated, aliphatic (straight or branched chains), alicyclic, or aromatic hydrocarbons. Suitable gelling agents for the anhydrous hydrocarbon gel include materials capable of thickening the hydrocarbon solvent. Suitable hydrocarbon gels are available under the trade name Versagel by the Penereco Corporation.

The translucent base layer may comprise a silicone gel. The silicone gels typically comprise a fluid silicone solvent and a silicone elastomer as a gelling agent. An example of a suitable silicone gel is Dow Coming 9040, which is a blend of cyclopentasiloxane and dimethicone crosspolymer.

The weight percentage, by weight of the base phase, of the solvent and gelling agent may vary based upon the end use of the skin care composition. The base phase may comprise from about 60 to about 99.8% solvent and from about 0.20% to about 15% gelling agent. The ratio of solvent to gelling agent is dictated by the desired viscosity of the base phase. In certain embodiments, the viscosity of the base phase is ideally greater than about 30,000 cps as measured by the Viscosity Method. In other embodiments, the viscosity of the base phase is ideally greater than about 40,000 cps.

The translucency of the base phase allows a consumer to fully appreciate the multi-phase character of the skin care composition by providing a visual signal to the consumer of presence of a distinct phase. The translucent base phase is sufficiently clear such that the pattern of one or more opaque phases may be discerned. In the absence of a translucent phase, the visually distinct pattern of the skin care composition may be underappreciated or unappreciated by a consumer. Furthermore, the translucency of the base phase allows the visually distinct pattern to be a three dimensional pattern. Without a translucent base layer (*i.e*., multiple opaque layers), three dimensionality would be lost. The base phase may be colored; however, the color of the translucent base layer should not interfere with the ability to discern the pattern of one or more opaque phases. Suitable colorants for the translucent base layer include any of the FD&C or D&C dyes. The translucent base phase ideally comprises less than about 0.05%, by weight of the base phase, of an opacifying particulate material. Greater than 0.05% opacifying particulate material may adversely impact the translucency of the base phase.

The translucent base phase may comprise one or more chronic skin care actives. As used herein, "chronic skin care active" means a compound useful for improving skin condition wherein the benefits are not realized at the time of application. The benefits of improved skin condition can include reduction of visibly and tactilely perceptible manifestations, as well as any macro- or micro-effects, due to keratinous tissue aging. Exemplary skin conditions include, but are not limited to, textural discontinuities such as wrinkles and coarse deep wrinkles, fine lines, skin lines, crevices, bumps, large pores, or unevenness; loss of skin elasticity; discoloration (including undereye circles); blotchiness; sallowness; hyperpigmented skin regions such as age spots and freckles; keratoses; abnormal differentiation; hyperkeratinization; elastosis; collagen breakdown, and other histological changes in the stratum corneum, dermis, epidermis, vascular system (*e.g*., telangiectasia or spider vessels), and underlying tissues (*e.g*., fat and/or muscle), especially those proximate to the skin. Usually the benefit of a chronic skin care active is achieved and/or perceived hours, days, or weeks after application. A chronic skin care active may require routine application in order for the benefit to be achieved and/or perceived. Chronic skin actives exclude skin conditioning agents and masking agents (*e.g*., titanium dioxide; iron oxides; matting particles including polyethylene, nylon, polymethylsilsesquioxane, and acrylic acid copolymers; interference pigments, or any other compound that provides an immediate change to skin appearance upon application).

Suitable chronic skin care actives include, but are not limited to, vitamins, peptides, sugar amines, sunscreens, oil control agents, tanning actives, anti-acne actives, desquamation actives, anti-cellulite actives, chelating agents, skin lightening agents, flavonoids, protease inhibitors, non-vitamin antioxidants and radical scavengers, hair growth regulators, anti-wrinkle actives, anti-atrophy actives, minerals, phytosterols and/or plant hormones, tyrosinase inhibitors, anti-inflammatory agents, N-acyl amino acid compounds, antimicrobials, and antifungals. These chronic skin care actives are provided in further detail in U.S. application publication No. US2006/0275237A1 and US2004/0175347A1.

Particularly suitable chronic skin actives include vitamin B3 compounds, sugar amines, peptides, and hexamidine. As used herein, "vitamin B₃ compound" means a compound having the formula: 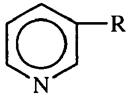 wherein R is - CONH₂ (*i.e*., niacinamide), - COOH (*i.e.*, nicotinic acid) or - CH₂OH (*i.e.,* nicotinyl alcohol); derivatives thereof; and salts of any of the foregoing. As used herein, "sugar amine" includes isomers and tautomers of such and its salts (*e.g*., HCl salt) and its derivatives. Examples of sugar amines include glucosamine, N-acetyl glucosamine, mannosanine, N-acetyl mannosamine, galactosamine, N-acetyl galactosamine, their isomers (*e.g.*, stereoisomers), and their salts (*e.g*., HCl salt). As used herein, "peptide" refers to peptides containing ten or fewer amino acids and their derivatives, isomers, and complexes with other species such as metal ions (*e.g.*, copper, zinc, manganese, magnesium, and the like). The compositions of the present invention can include hexamidine compounds, its salts, and derivatives. As used herein, "hexaminide compound" means a compound having the formula: 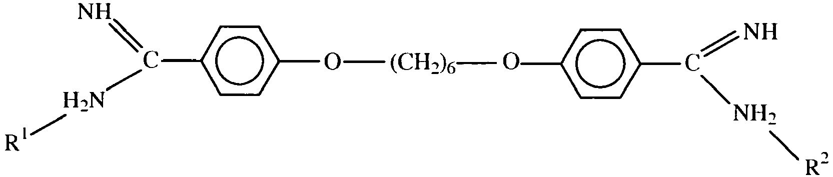 wherein R¹ and R² are optional or are organic acids (*e.g*., sulfonic acids, etc.).

First Opaque Phase - The first opaque phase may be in any conventional form such as a emulsion or a gel (*e.g*., an aqueous gel, an anhydrous hydrocarbon gel, or an anhydrous silicone gel). In certain embodiments, the first opaque phase is in the form of an emulsion. Emulsions may be generally classified as having a continuous aqueous phase (*e.g*., oil-in-water and water-in-oil-in-water) or a continuous oil phase (*e.g*., water-in-oil and oil-in-water-in-oil). The oil phase of the present invention may comprise silicone oils, non-silicone oils such as hydrocarbon oils, esters, ethers, and the like, and mixtures thereof. For example, suitable emulsions include, but are not limited to, continuous water phase emulsions such as silicone-in-water, oil-in-water, and water-in-oil-in-water emulsion; and continuous oil phase emulsions such as water-in-oil and water-in-silicone emulsions, and oil-in-water-in-silicone emulsions. The aqueous phase typically comprises water. However, the aqueous phase may comprise non-water carriers such as glycerin or other polyols.

Emulsions may further comprise an emulsifier. The skin care composition may comprise from about 0.1% to about 10% or about 0.2% to about 5% of an emulsifier, based on the weight of the composition. Emulsifiers may be nonionic, anionic or cationic. Suitable emulsifiers are disclosed in, for example, U.S. Patent 3,755,560, U.S. Patent 4,421,769, and McCutcheon's Detergents and Emulsifiers, North American Edition, pages 317-324 (1986).

The first opaque phase may further comprise a thickening agent to yield a suitable viscosity and rheological character. If the first opaque phase is a water continuous emulsion, suitable thickening agents include xanthan gum, polysaccharides, carbomers, and polymers and copolymers of acrylic acid, acylamide, acryloyldimethyl taurate and their derivatives. Suitable thickening agents include Sepigel 305, Simulgel INS-100, Simulgel SMS 88, and Simulgel EG; all available from Seppic, Inc. In certain embodiments, the viscosity of the first opaque phase is ideally greater than about 30,000 cps as measured by the Viscosity Method. In other embodiments, the viscosity of the first opaque phase is ideally greater than about 40,000 cps.

The opacity of the first opaque phase allows a consumer to fully appreciate the multi-phase character of the skin care composition. When paired with the translucent base phase, the skin care composition may exhibit a visually distinct pattern which may be a three dimensional pattern. The first opaque phase may exhibit a color providing a visual signal to the consumer of the presence of a distinct phase. The color may further provide a signal to the consumer of the function, benefit, or effect of the first opaque phase. In one embodiment, the first opaque phase exhibits a lightness (L) of greater than about 80 and a chroma (C) of less than about 5. In other embodiments, the first opaque phase exhibits a lightness (L) of greater than about 85 and a chroma (C) of less than about 3.5 or of less than about 2.

The white appearance of the first opaque phase may be provided by one or more opacifying particulate materials. Exemplary opacifying particulate materials include titanium dioxide, zinc oxide, zirconium dioxide, and the like. In certain embodiments, the first opaque phase may comprise greater than about 0.05%, by weight of the first opaque phase, of one or more opacifying particulate materials. Titanium dioxide is a particularly suitable opacifying particulate material.

In other embodiments, the first opaque phase is substantially or entirely free of opacifying particulate materials. Less than 0.05%, by weight of the first opaque phase, of opacifying particulate materials is not believed to significantly impact the opacity of the phase. In such embodiments, the opacity and color of the first opaque phase are a result of the emulsion that forms the first opaque phase.

The first opaque phase may comprise one or more skin conditioning agents. The skin conditioning agent is useful for lubricating the skin, increasing the smoothness and suppleness of the skin, preventing or relieving dryness of the skin, hydrating the skin, and/or protecting the skin. The skin conditioning agent is preferably selected from the group consisting of emollients, humectants, and mixtures thereof. The skin conditioning agent is may be present in the first opaque phase at a level of at least about 0.1%, about 1.0%, about 2.0%, or about 5.0% and at a level of no more than about 99%, about 50%, about 30%, or about 25%. A variety of emollients may be employed. Suitable emollients are designated as "Skin-Conditioning Agents - Emollient" in the Cosmetic, Toiletry, and Fragrance Association (CTFA) International Cosmetic Ingredient Dictionary and Handbook, 11th ed*.* Suitable humectants include polyols such as polyalkylene glycols, sorbitol, hydroxypropyl sorbitol, erythritol, threitol, pentaerythritol, xylitol, glucitol, mannitol, hexylene glycol, butylene glycol, hexane triol, glycerol, ethoxylated glycerol, propoxylated glycerol, and other compounds designated as "Skin-Conditioning Agents - Humectant" in the Cosmetic, Toiletry, and Fragrance Association (CTFA) International Cosmetic Ingredient Dictionary and Handbook, 11th ed*.*

Second Opaque Phase - The second opaque phase may be in any conventional form such as an emulsion or a gel (*e.g.,* an aqueous gel, an anhydrous hydrocarbon gel, or an anhydrous silicone gel). In certain embodiments, the first opaque phase is in the form of an emulsion. Emulsions may be generally classified as having a continuous aqueous phase (e.g., oil-in-water and water-in-oil-in-water) or a continuous oil phase (e.g., water-in-oil and oil-in-water-in-oil). The oil phase of the present invention may comprise silicone oils, non-silicone oils such as hydrocarbon oils, esters, ethers, and the like, and mixtures thereof. For example, suitable emulsions include, but are not limited to, continuous water phase emulsions such as silicone-in-water, oil-in-water, and water-in-oil-in-water emulsion; and continuous oil phase emulsions such as water-in-oil and water-in-silicone emulsions, and oil-in-water-in-silicone emulsions. The aqueous phase typically comprises water. However, the aqueous phase may comprise non-water carriers such as glycerin or other polyols.

Emulsions may further comprise an emulsifier. The skin care composition may comprise from about 0.1% to about 10% or about 0.2% to about 5% of an emulsifier, based on the weight of the composition. Emulsifiers may be nonionic, anionic or cationic. Suitable emulsifiers are disclosed in, for example, U.S. Patent 3,755,560, U.S. Patent 4,421,769, and McCutcheon's Detergents and Emulsifiers, North American Edition, pages 317-324 (1986).

The second opaque phase may further comprise a thickening agent to yield a suitable viscosity and rheological character. If the second opaque phase is a water continuous emulsion, suitable thickening agents include xanthan gum, polysaccharides, carbomers, and polymers and copolymers of acrylic acid, acylamide, acryloyldimethyl taurate and their derivatives. Suitable thickening agents include Sepigel 305, Simulgel INS-100, Simulgel SMS 88, and Simulgel EG; all available from Seppic, Inc. In certain embodiments, the viscosity of the second opaque phase is ideally greater than about 30,000 cps as measured by the Viscosity Method. In other embodiments, the viscosity of the second opaque phase is ideally greater than about 40,000.

The opacity of the second opaque phase allows a consumer to fully appreciate the multi-phase character of the skin care composition. When paired with the translucent base phase, the skin care composition may exhibit a visually distinct pattern which may be a three dimensional pattern. The second opaque phase may exhibit a color that providing a visual signal to the consumer of presence of a phase distinct from the translucent base phase and the first opaque phase. The color may further provide a signal to the consumer of the function, benefit, or effect of the second opaque phase. In one embodiment, the second opaque phase exhibits a hue (H) of between about 35 to about 70. In other embodiments, the second opaque phase exhibits a hue (H) between about 45 to about 65. Furthermore, the second opaque phase may exhibit a lightness (L) of between about 35 and about 80 and a chroma (C) of between about 15 and about 30. Alternatively, the second opaque phase may exhibit a lightness (L) of between about 40 and about 75 and a chroma (C) of between about 18 and about 28.

The color of the second opaque phase may be provided by one or more opacifying particulate materials and/or one or more colorants. Exemplary opacifying particulate materials include titanium dioxide, zinc oxide, zirconium dioxide, and the like. In certain embodiments, the second opaque phase may comprise greater than about 0.3%, by weight of the second opaque phase, of one or more opacifying particulate materials. In other embodiments, the second opaque phase may comprise greater than about 3% or about 5%, by weight of the second opaque phase, of one or more opacifying particulate materials. Titanium dioxide is a particularly suitable opacifying particulate material. Colorants for use in the second opaque phase may be selected from the group consisting of organic pigments, inorganic pigments, interference pigments, lakes, natural colorants, pearlescent agents, dyes (including, for example, water-soluble, non-soluble, oil-soluble), carmines, and mixtures thereof. In certain embodiments, the second opaque phase may comprise greater than about 0.3%, by weight of the second opaque phase, of one or more colorants. In other embodiments, the second opaque phase may comprise greater than about 0.5% or about 1%, by weight of the second opaque phase, of one or more colorants. In certain embodiments, it may be desirable that the second opaque phase contains both an opacifying particulate material and a colorant.

Other Optional Actives - In certain embodiments, the chronic skin care active of the translucent base phase, the skin conditioning agent of the first opaque phase, and the opacifying material of the second opaque phase are present only in said phase. In other embodiments, it should be recognized that the chronic skin care active, the skin conditioning agent, and opacifying material may be present in more than one phase. For example, the chronic skin care active of the base translucent phase may be present in the first and second opaque phases. Alternatively, the translucent phase may comprise a first chronic skin care active and the first and/or second opaque phases may comprise a second chronic skin care active different from the first skin care active.

One or more phases may comprise sunscreen actives. Suitable sunscreen actives include oil-soluble sunscreens, insoluble sunscreens, and water-soluble sunscreens. In certain embodiments, the phase may comprise from about 10% to about 50%, by weight of the phase, of one or more sunscreen actives. In other embodiments, the phase may comprise from about 10% to about 40% or from about 15% to about 30%, by weight of the phase, of one or more sunscreen actives. In certain embodiments, the translucent base phase is substantially free of sunscreen actives. Exact amounts will vary depending upon the chosen sunscreen active and/or ultraviolet light absorber and the desired Sun Protection Factor (SPF), and are within the knowledge and judgment of one of skill in the art.

Non-limiting examples of suitable oil-soluble sunscreens include benzophenone-3, bis-ethylhexyloxyphenol methoxyphenyl triazine, butyl methoxydibenzoyl-methane, ethylhexyl methoxy-cinnamate, ethylhexyl salicylate, ethylhexyl triazone, octocrylene, homosalate, and derivatives and mixtures thereof. Non-limiting examples of suitable insoluble sunscreens include methylene bis-benzotriazolyl tetramethylbutyl-phenol, titanium dioxide, zinc oxide, and derivatives and mixtures thereof. Non-limiting examples of suitable water-soluble sunscreens include phenylbenzimidazole sulfonic acid (PBSA), terephthalylidene dicamphor sulfonic acid (Mexoryl™ SX), and salts, derivatives and mixtures thereof. Other suitable sunscreens are designated as "Sunscreen Agents" or "Ultraviolet Light Absorbers" in the Cosmetic, Toiletry, and Fragrance Association (CTFA) *International Cosmetic Ingredient Dictionary and Handbook*, *11^{th} ed.*

Container - The skin care product further comprises a container with an interior space in which the skin care composition is housed. The container may be partitioned such that the container comprises multiple interior compartments that are physically separated from one another. However, the multi-functional skin care composition of the present invention involves at least three phases that occupy separate spaces with a container wherein each phase is in contact with at least one other phase (*i.e.,* the phases are not stored in separate compartments or partitions in the container).

While the specific size, shape, and materials of the container are not specifically limited, the container should be at least partially translucent in order to display the visually distinct pattern of the skin care composition housed therein. A partially translucent container may be a container that is mostly opaque but comprises a translucent window through which the skin care composition may be viewed. The translucent window should be constructed such that the visually distinct pattern of the skin care composition can be appreciated by viewer. A partially translucent container may be a container that is constructed such that the visually distinct pattern of the skin care composition can be viewed through a majority of the exterior surface area of the container.

The container may be constructed of a flexible, semi-rigid, or rigid material. In embodiments where the skin care composition forms a visually distinct repeating pattern that is uniform in appearance (*i.e.*, not a random pattern), the container may preferably be constructed from a material that does not substantially deform or flex under a typical pinch force exerted between the fingers and thumb, which is approximately 5 pounds force. A container constructed from a material that does not substantially deform or flex protects the visually distinct repeating pattern from being disturbed or misshapen.

In embodiments where the skin care composition forms a visually distinct repeating pattern that is uniform in appearance (*i.e*., not a random pattern), a container having a uniform interior space may be preferred such as a container having a cylindrical interior space.

The container further comprises a means for dispensing, delivering, or accessing the skin care composition. For example, the container may include a threaded opening removably covered by a threaded lip (*e.g*., a screw top jar) where the skin care composing is removed by the user through the opening. In another example, the container may include a dispensing mechanism that dispenses a portion of the skin care composition upon activation. A suitable dispensing mechanism is a pump, which is well known and commercially available from various suppliers. Another suitable dispensing mechanism is piston that applies force either directly or indirectly to the skin care composition thereby expelling the skin care composition from an orifice in the container. Suitable piston-type dispensing mechanisms include the mechanisms of U.S. Patent No. 5,961,007 and PCT Publication No. WO 2006/075484.

It should be recognized that, in many embodiments of the present invention, the second opaque phase may function as a make-up product that provides an acute skin tone benefit such as evening skin tone or masking imperfections. The specific components providing the acute skin tone benefit (*e.g*., opacifying agents and colorants) may be present only in the second phase and may give visual distinctiveness to the second phase. However, upon dispensing, the skin care composition, it may be desirable for the composition to have a homogenous appearance. The term "homogenous appearance" means that the phases of the composition can not be seen by the human eye as distinctly separate regions. A skin care composition dispensed without a homogenous appearance is generally undesirable because it requires user involvement to mix the composition. Otherwise, if applied to the skin without user mixing, the composition may give the skin an unacceptable mottled or streaked appearance. In light of this problem, a dispensing mechanism should dispense the multi-phase skin care composition as a visually homogenous pattern. A suitable homogenizing dispensing mechanism and container are Mega Micro 0.25 mL dose pumphead along with a 50mL micro round transparent glossy container (#AA0KAA00022)) available from MegaPlast GmbH, Germany. In certain embodiments, the dispensing mechanism yields the care composition in a post-dispense form wherein the post-dispensed skin care composition exhibits a hue of from about 35 to about 70.

The skin care product may further comprise a display package. The display package is any packaging that partially or fully houses the container. Suitable display packages include boxes, plastic clamshell packaging, film and foil overwraps, blister packs, plastic trays, pillow packs, buckets, end sealed and/or capped tubes, and the like. The display package may be at least partially translucent such that at least a portion of the container can be seen and, ideally, the visually distinct pattern of the skin care composition housed therein may be seen. The display package may contain graphics, trademarks, brand identifiers, advertising claims, product benefit statements, and the like.

Informational Indicia - The skin care product of the present invention may comprise informational indicium or indicia. Informational indicia include any graphic, symbol, icon, word, phrase, or other marking that communicates a function or benefit of the skin care product to a user. The information indicia may be disposed on the container, the display packaging, or on other printed materials that are associated with or accompany the skin care product. For example, a skin care product that functions as a sunscreen may have the textual informational indicium such as "Protects skin from the sun" or may have a graphic informational indicium such as a line drawing of the sun.

In many cases, a skin care product may include several informational indicium. For a multi-functional skin care composition it may be desirable to provide an indicium for each of the functions of the composition. For a multi-phase skin care composition, it may be desirable to provide an indicium for each of the phases in the composition. In certain embodiments, an information indicium communicates the function or benefit of a particular phase within the skin care composition. In an embodiment comprising a skin care compositing with a translucent base phase with a chronic skin care active, a first opaque phase with a skin conditioning agent, and a second opaque phase with an opacifying particulate material, the informational indicia may include the phrases: "gradual repair of skin imperfections", "immediate moisturization of skin", and "conceals imperfections of the skin." Alternatively, the same embodiment may comprise information indicia including a before and after photograph or graphic depicting wrinkle reduction, a photograph or graphic depicting water or a water drop, and a before and after photograph or graphic depicting evening of skin tonality. It should be recognized that the exact phrasing of the textual informational or visual representation of a graphic, symbol, or icon may vary for a particular function or benefit.

Commercial Array - Another aspect of the present invention relates to a commercial array of skin care products. The term "commercial array" means a two or more products that are (i) manufactured by a single entity or (ii) distributed by a single entity. The products of the commercial array may include a common designator such as a marking of a common distributor, a marking of a common manufacturer, a common trademark, or common trade dress.

The present invention relates to a commercial array of skin care products comprising at least a first skin care product and a second skin care product. The first skin care product has a first non-solid multi-phase multi-functional skin care composition and a first container for storing the multi-phase skin care composition, and the second skin care product has a second non-solid multi-phase multi-functional skin care composition and a second container for storing the multi-phase skin care composition. The non-solid multi-phase multi-functional skin care composition of the first and second skin care products may each comprise a translucent base phase, a first opaque phase having a lightness of greater than about 80 and a chroma of less than about 5; and a second opaque phase having a lightness of less than about 80 and a chroma of greater than about 15. The translucent base phase, first opaque phase, and second opaque phase form a stable, visually distinct pattern. The first multi-phase skin care composition and the second multi-phase skin care composition differ in the visual appearance of a analogous layer such as the translucent base phase, the first opaque phase, or the second opaque phase. In one embodiment, the second opaque phase of the first multi-phase skin care composition and the second opaque phase of the second multi-phase skin care composition exhibit a lightness difference (ΔL) by at least 4. The lightness difference (ΔL) is calculated by taking the absolute value of the difference of the L value of the second opaque phase of the first multi-phase skin care composition and the L value of the second opaque phase of the second multi-phase skin care composition. In other embodiments, the second opaque phase of the first multi-phase skin care composition and the second opaque phase of the second multi-phase skin care composition exhibit a lightness difference (ΔL) by at least 8.

### Test Methods

Sample Preparation - For the following test methods, samples are taken with reasonable care. When the viscosity or color value of an individual phase is being characterized, the individual phase is to be separated from the skin care composition. The individual phase is to be separated such that no other phases can be seen within the sample tested. The individual phase is to be separated to avoid unnecessary mixing or disruption of the phases. It should be recognized that sample preparation may require dispensing the skin care composition in a non-standard manner. Dispensing the skin care composition may require the irreversible opening of the container such as by bisecting the container or other breach of the container in a manner to access the skin care composition without unnecessary mixing or disruption of the phases.

Viscosity Method - Viscosities are measured on a Brookfield viscometer using a T-C bar spindle with a heliopath setting at 5 rpm at 25°C

Colorimetery Method - Lightness, chorma, and hue are measured by creating a thick film of the sample (using a 10 mils Wet Film Thickness Byrd applicator on the black portion of Leneta Form 2A Opacity Chart) and measuring the D65/10 CIELAB color values of this film with a D/8 geometry integrating sphere spectrophotometer, measuring spectral excluded. A particularly preferred colorimeter is the Microflash^{®} spectophotometer available from Datacolor Int'l, Charlotte, NC (such as the Microflash^{®} 200d).

### Examples

Examples A-E are exemplary individual phases that may be combined to form the multi-phase skin care composition of the present invention

| Ingredient (Wt%) | Phase A | Phase B | Phase C | Phase D | Phase E |
|---|---|---|---|---|---|
| Octyl Salicylate | | 3.0000 | 10.00 | 10.00 | 10.00 |
| Avobenzone | | 2.0000 | 6.67 | 6.67 | 6.67 |
| Octocrylene | | 3.0000 | 10.00 | 10.00 | 10.00 |
| Water (Aqua) | QS | QS | QS | QS | QS |
| TiO₂ Predispersion ^{*A} | | | 15.143 | 17.760 | 22.619 |
| Glycerin | 7.00 | 7.000 | 7.00 | 7.00 | 7.00 |
| Niacinamide | 4.00 | 4.000 | 4.00 | 4.00 | 4.00 |
| DC 1503 ^{*B} | | 2.000 | 2.00 | 2.00 | 2.00 |
| N-Acetyl Glucosamine | 2.00 | 2.000 | 2.00 | 2.00 | 2.00 |
| Isopropyl N-Lauroylsarcosinate | | 2.000 | 10.00 | 10.00 | 10.00 |
| Yellow Iron Oxide Predispersion ^{*C} | | | 5.664 | 4.3290 | 2.923 |
| Sepigel 305 ^{*D} | | 1.000 | 0.50 | 0.50 | 0.500 |
| Red Iron Oxide Predispersion ^{*E} | | | 1.693 | 1.332 | 0.981 |
| Triethanolamine | 0.55 | | | | |
| Cetyl alcohol | | 0.500 | 0.60 | 0.60 | 0.60 |
| Panthenol | 0.50 | 0.500 | 0.50 | 0.50 | 0.50 |
| Carbomer ^{*F} | 0.50 | | | | |
| Behenyl alcohol | | 0.500 | 1.00 | 1.00 | 1.00 |
| Stearyl alcohol | | 0.500 | 0.90 | 0.90 | 0.90 |
| Benzyl Alcohol | 0.25 | 0.500 | 0.50 | 0.50 | 0.500 |
| Tocopheryl Acetate | | 0.200 | 0.670 | 0.670 | 0.670 |
| Emulgade PL 68/50 ^{*G} | | 0.200 | 0.50 | 0.50 | 0.500 |
| Ethylparaben | 0.10 | 0.200 | 0.200 | 0.200 | 0.20 |
| Methylparaben | 0.10 | 0.200 | 0.200 | 0.200 | 0.20 |
| Black Iron Oxide Predispersion ^{*H} | | | 0.599 | 0.3770 | 0.278 |
| PEG-100 stearate | | 0.100 | 0.250 | 0.250 | 0.25 |
| Propylparaben | | 0.100 | 0.100 | 0.100 | 0.10 |
| Glycasil L ^{*I} | | 0.100 | 0.100 | 0.100 | 0.10 |

| | | | | | |
|---|---|---|---|---|---|
| A - Supplier code GLW65TAP available from Kobo Products, Inc, South Plainfield, NJ. B - Dimethicone and dimethiconol mixture available from Dow Coming, Inc, Midland, MI. C - Supplier code GLW45GYAP available from Kobo Products, Inc, South Plainfield, NJ. D - Polyacrylamide, C13-14 isoparaffin, laureth-7, and water mixture available from Seppic, Inc., Fairfield, NJ. E - Supplier code GLW55GYAP available from Kobo Products, Inc, South Plainfield, NJ. F - Ultrez 10 available from Noveon, Inc., Cleveland, OH. G - Cetearyl glucoside and cetearyl alcohol mixture available from Cognis, Cincinnati, OH. H - Supplier code GLW60GBAP available from Kobo Products, Inc, South Plainfield, NJ. I - Polyethylene glycol monococoate, polyethylene glycol dicocoate, idopropynyl butyl carbamate, and polyethylene glycol mixture from Lonza, Inc., Allendale, NJ. | | | | | |

Phase A is prepared by mixing the ingredients together in a suitable vessel. Phase A yields a translucent aqueous gel.

Phases B-E are prepared by first mixing the water phase materials together in a suitable vessel with gradual heating to 80°C. The oil phase materials are mixed in a separate suitable vessel with gradual heating to 80°C. When both the water and oil phase materials reach 80°C, the oil phase is slowly added to the water phase with continuous milling of the system to form an emulsion (*i.e.*, oil-in-water).

Phase B yields an opaque phase that is white in color. Phase B exhibits a lightness of greater than 80 and a chroma of less than 5. Specifically, Phase B exhibits a lightness of 90 and a chroma of 3.

Phase C yields an opaque phase that is beige in color. Phase C exhibits a hue from about 35 to about 70. Specifically, Phase C exhibits a hue of 58. Phase C exhibits a lightness of 60 and a chroma of 26.

Phase D yields an opaque phase that is beige in color. Phase D exhibits a hue from about 35 to about 70. Specifically, Phase D exhibits a hue of 57. Phase D exhibits a lightness of 65 and a chroma of 24.

Phase E yields an opaque phase that is beige in color. Phase E exhibits a hue from about 35 to about 70. Specifically, Phase E exhibits a hue of 58. Phase E exhibits a lightness of 70 and a chroma of 22.

Suitable skin care products having a multi-phase skin care composition with a stable, visually distinct pattern may be formed with the following combination of phases (Phases A/B/C, Phases A/B/D, and Phases A/B/E) in a ratio of 60:10:30. The container for each of the previous combination was Mega Micro 0.25 mL dose pumphead and a 50mL micro round transparent glossy container (#AA0KAA00022) available from MegaPlast GmbH, Germany. The skin care product of the present invention may be prepared by any known or otherwise effective technique, suitable for making and formulating the desired non-solid multi-phase skin care composition. The present invention can be prepared by the method and apparatus as disclosed in U.S. Pat. No. 6,213,166. The method and apparatus allows two or more compositions or phases to be filled with a spiral configuration into a single container. The method requires that at least two nozzles be employed to fill the container. The container is placed on a rotating mixer and spun as the composition is introduced into the container.

A suitable array of skin care products comprising at least a first skin care product and a second skin care product was created in the preceding paragraph with the combination of phases: Phases A/B/C, Phases A/B/D, and Phases A/B/E. With regard to Phases A/B/C and Phases A/B/D, Phases C and D exhibit a lightness difference (ΔL) of 5. With regard to Phases A/B/C and Phases A/B/E, Phases C and E exhibit a lightness difference (ΔL) of 10.

### Consumer Perception Study

The skin care composition comprising multiple phases in a visually distinct pattern has been found to be an important factor in communicating the multi-functionality of the skin care composition. A study was performed comparing two skin care compositions. The first composition (Composition I) was a multi-phase product, which represents one embodiment of the present invention, comprising the Phases A/B/D (in a 60:10:30 ratio) as described in the Example section above. The second composition (Composition II) was a dual phase product, which is not within the present invention, comprising Phases A/D (in a 70:30 ratio) as described in the Example section above. The visually distinct pattern was similar in both compositions except for the absence of Phase B in the second composition. Compositions I and II were contained within identical containers that were substantially transparent. Compositions I and II were visually inspected by 61 panelists. Panelists could visually observe the skin care products but were not allowed to dispense the skin care composition or obtain additional sensory information except for sight. The panelists answered various questions about Compositions I and II according to a qualitative scale (e.g., not at all=0, not very effective=25, somewhat effective=50, very effective=75, extremely effective=100). Answers were statistically analyzed and a mean score provided (p=0.05). Provided below are the results of two questions relating to the functionality or benefit of the Compositions.

| **Question** | **Composition I** | **Composition II** |
|---|---|---|
| Perceived moisturization effectiveness | 75 | 64 |
| Perceived anti-aging effectiveness | 72 | 66 |
| Reduces the need for make-up/foundation | 70 | 70 |

The panelists' answers to the first and second questions indicate that the presence of Phase B which is visually distinct from Phases A and D increases the perceived moisturization and anti-aging effectiveness of the composition. In follow-up study, 27 of the 61 panelists were invited to use Composition I and Composition II. After inspecting and using the Compositions, a significant majority of the interviewees (*i.e.*, 16 to 4) perceived Composition I as being more indicative of an anti-aging moisturizer versus Composition II. It should be recognized that the skin conditioning agent providing the moisturization benefit (*e.g*., glycerine) is present at an equivalent weight percentage in Compositions I and II. Even with both Compositions having an equivalent amount of moisturizing active (*e.g*., glycerine), the 3-phased Composition I is perceived as indicative of a moisturizer after use.

The consumer perception study evidences that skin care compositions having multiple visually distinct phases more effectively convey multi-functionality. While not wishing to be bound by theory, it is believed that the particular appearance of the phase signals or conveys the function or benefit of the phase. Phase D exhibits a hue of 57 which conveys the benefit of a make-up, a cosmetic foundation, or concealer. Phase D provides immediate skin tone adjustment which is common to make-up and cosmetic products. The benefit can be associated with the opacifying particulate materials and/or the colorants within the phase. Phase A is transparent and is believe to convey a chronic benefit such as anti-aging or wrinkle reduction which can be associated with the chronic skin care agents within the phase. Phase B exhibits a lightness of greater than about 80 and a chroma of less than about 5, and appears to be white in color. Phase B is believe to convey a skin conditioning benefit such as moisturization or softening which is associated with the skin conditioning agents within the phase. The unexpected result of the Consumer Perception Study is that the addition of a third visually distinct phase conveys improved multi-functionality compared to a skin care composition having two visually distinct phases. The particular lightness, chroma, hue, and/or translucency of each layer may further convey the multi-functionality of the skin care composition.

## Claims

1. A skin care product comprising:
a) a non-solid multi-phase, multi-function skin care composition comprising:
(i) a translucent base phase comprising a first chronic skin care active;
(ii) a first opaque phase having a lightness of greater than 80 and a chroma of less than 5, preferably a lightness of greater than 85 and a chroma of less than 2; wherein said first opaque phase comprises a skin conditioning agent; and
(iii) a second opaque phase having a hue from 35 to 70, said second opaque phase comprising an opacifying particulate materials;
wherein the translucent base phase, first phase, and second phase form a stable, visually distinct pattern, and the lightness, chroma and hue are measured according to the method described in the description; and
b) a container for storing the multi-phase skin care composition, wherein the container is at least partially translucent.

2. The skin care product of Claim 1 wherein said first chronic skin care active is selected from sugar amines, B3 compounds, peptides, hexamidine compounds, and mixtures thereof.

3. The skin care product according to any one of the preceding claims wherein the first opaque phase further comprises from 15% to 30%, by weight of the first opaque phase, of one or more sunscreen actives.

4. The skin care product according to any one of the preceding claims wherein the second opaque phase further comprises a second chronic skin care active.

5. The skin care product according to any one of the preceding claims wherein the skin care composition comprises 55% to 75% of the translucent base phase; 5 to 15% of the first opaque phase; and 20 to 30% of the second opaque phase.

6. The skin care product according to any one of the preceding claims wherein the translucent base phase is an aqueous gel and wherein the first opaque phase and the second opaque phase are oil-in-water emulsions.

7. The skin care product according to any one of the preceding claims wherein the second opaque phase has:
a) a lightness from aout 40 to 75;
b) a chroma of from 18 to 28; and
c) a hue from 45 to 65.

8. The skin care product according to any one of the preceding claims wherein the translucent base phase, first opaque phase, and second opaque phase each exhibit a viscosity of greater than 40,000 cps.

9. The skin care product according to any one of the preceding claims wherein the container further comprises a dispensing mechanism for dispensing the multi-phase skin care composition, wherein the post-dispensed skin care composition exhibits a homogenous appearance.

10. The skin care product of Claim 11 wherein post-dispensed skin care composition exhibits a hue of from 35 to 70.

11. An array of skin care products comprising a first skin care product and a second skin care product, wherein
a) the first skin care product comprises
(i) a first non-solid multi-phase, multi-function skin care composition comprising:
(a) a translucent base phase
(b) a first opaque phase having a lightness of greater than 80 and a chroma of less than 5; and
(c) a second opaque phase having a hue from 35 to 70 and a lightness value;
wherein the translucent base phase, first phase, and second phase form a stable, visually distinct pattern, and the lightness, chroma and hue are measured according to the method described in the description; and
(ii) a first container for storing the multi-phase skin care composition, wherein the container is at least partially translucent; and
b) the second skin care product comprises
(i) a second non-solid multi-phase, multi-function skin care composition comprising:
(a) a translucent base phase
(b) a first opaque phase having a lightness of greater than 80 and a chroma of less than 5; and
(c) a second opaque phase having a hue from 35 to 70 and a lightness value;
wherein the translucent base phase, first phase, and second phase form a stable, visually distinct pattern, the lightness, chroma and hue are measured according to the method described in the description; and
(ii) a second container for storing the multi-phase skin care composition, wherein the container is at least partially translucent;
wherein the second opaque phase of the first multi-phase skin care composition and the second opaque phase of the second multi-phase skin care composition exhibit a lightness difference (ΔL) of at least 4.

## Patentansprüche

1. Hautpflegeprodukt, umfassend:
a) eine nicht feste, mehrphasige Multifunktions-Hautpflegezusammensetzung, umfassend:
(I) eine durchsichtige Grundphase, umfassend einen ersten chronischen Hautpflegewirkstoff,
(II) eine erste undurchsichtige Phase mit einer Helligkeit von mehr als 80 und einer Farbsättigung von weniger als 5, vorzugsweise einer Helligkeit von mehr als 85 und einer Farbsättigung von weniger als 2,
wobei die erste undurchsichtige Phase ein Hautkonditioniermittel umfasst, und
(III) eine zweite undurchsichtige Phase mit einem Farbton von 35 bis 70, wobei die zweite undurchsichtige Phase trübende teilchenförmige Materialien umfasst,
wobei die durchsichtige Grundphase, die erste Phase und die zweite Phase ein stabiles, optisch unterscheidbares Muster bilden und die Helligkeit, die Farbsättigung und der Farbton gemäß dem in der Beschreibung beschriebenen Verfahren gemessen werden, und
b) einen Behälter zur Aufbewahrung der mehrphasigen Hautpflegezusammensetzung, wobei der Behälter wenigstens teilweise durchsichtig ist.

2. Hautpflegeprodukt nach Anspruch 1, wobei der erste chronische Hautpflegewirkstoff ausgewählt ist aus Zuckeraminen, B3-Verbindungen, Peptiden, Hexamidin-Verbindungen und Mischungen davon.

3. Hautpflegeprodukt nach einem der vorstehenden Ansprüche, wobei die erste undurchsichtige Phase ferner zu 15 % bis 30 %, bezogen auf das Gewicht der ersten undurchsichtigen Phase, einen oder mehrere Sonnenschutzwirkstoffe umfasst.

4. Hautpflegeprodukt nach einem der vorstehenden Ansprüche, wobei die zweite undurchsichtige Phase ferner einen zweiten chronischen Hautpflegewirkstoff umfasst.

5. Hautpflegeprodukt nach einem der vorstehenden Ansprüche, wobei die Hautpflegezusammensetzung zu 55 % bis 75 % die durchsichtige Grundphase, zu 5 bis 15 % die erste undurchsichtige Phase und zu 20 bis 30 % die zweite undurchsichtige Phase umfasst.

6. Hautpflegeprodukt nach einem der vorstehenden Ansprüche, wobei die durchsichtige Grundphase ein wässriges Gel ist und wobei die erste undurchsichtige Phase und die zweite undurchsichtige Phase Öl-in-Wasser-Emulsionen sind.

7. Hautpflegeprodukt nach einem der vorstehenden Ansprüche, wobei die zweite undurchsichtige Phase Folgendes aufweist:
a) eine Helligkeit von etwa 40 bis 75,
b) eine Farbsättigung von 18 bis 28 und
c) einen Farbton von 45 bis 65.

8. Hautpflegeprodukt nach einem der vorstehenden Ansprüche, wobei die durchsichtige Grundphase, die erste undurchsichtige Phase und die zweite undurchsichtige Phase jeweils eine Viskosität von mehr als 40.000 cP aufweisen.

9. Hautpflegeprodukt nach einem der vorstehenden Ansprüche, wobei der Behälter ferner einen Abgabemechanismus zum Abgeben der mehrphasigen Hautpflegezusammensetzung umfasst, wobei die Hautpflegezusammensetzung nach der Abgabe ein homogenes Erscheinungsbild aufweist.

10. Hautpflegeprodukt nach Anspruch 11, wobei die Hautpflegezusammensetzung nach der Abgabe einen Farbton von 35 bis 70 aufweist.

11. Reihe von Hautpflegeprodukten, umfassend ein erstes Hautpflegeprodukt und ein zweites Hautpflegeprodukt, wobei
a) das erste Hautpflegeprodukt Folgendes umfasst:
(I) eine erste nicht feste, mehrphasige Multifunktions-Hautpflegezusammensetzung, umfassend:
(a) eine durchsichtige Grundphase
(b) eine erste undurchsichtige Phase mit einer Helligkeit von mehr als 80 und einer Farbsättigung von weniger als 5 und
(c) eine zweite undurchsichtige Phase mit einem Farbton von 35 bis 70 und einem Helligkeitswert,
wobei die durchsichtige Grundphase, die erste Phase und die zweite Phase ein stabiles, optisch unterscheidbares Muster bilden und die Helligkeit, die Farbsättigung und der Farbton gemäß dem in der Beschreibung beschriebenen Verfahren gemessen werden, und
(II) einen ersten Behälter zur Aufbewahrung der mehrphasigen Hautpflegezusammensetzung, wobei der Behälter wenigstens teilweise durchsichtig ist, und
b) das zweite Hautpflegeprodukt Folgendes umfasst:
(I) eine zweite nicht feste, mehrphasige Multifunktions-Hautpflegezusammensetzung, umfassend:
(a) eine durchsichtige Grundphase,
(b) eine erste undurchsichtige Phase mit einer Helligkeit von mehr als 80 und einer Farbsättigung von weniger als 5 und
(c) eine zweite undurchsichtige Phase mit einem Farbton von 35 bis 70 und einem Helligkeitswert,
wobei die durchsichtige Grundphase, die erste Phase und die zweite Phase ein stabiles, optisch unterscheidbares Muster bilden und die Helligkeit, die Farbsättigung und der Farbton gemäß dem in der Beschreibung beschriebenen Verfahren gemessen werden, und
(II) einen zweiten Behälter zur Aufbewahrung der mehrphasigen Hautpflegezusammensetzung, wobei der Behälter wenigstens teilweise durchsichtig ist,
wobei die zweite undurchsichtige Phase der ersten mehrphasigen Hautpflegezusammensetzung und die zweite undurchsichtige Phase der zweiten mehrphasigen Hautpflegezusammensetzung einen Helligkeitsunterschied (• L) von mindestens 4 aufweisen.

## Revendications

1. Produit de soins pour la peau comprenant :
a) une composition de soins pour la peau non solide, à fonctions multiples et à phases multiples incluant :
(i) une phase de base translucide comprenant un premier agent actif chronique de soins pour la peau ;
(ii) une première phase opaque présentant une luminosité supérieure à 80 et une chrominance inférieure à 5, de préférence une luminosité supérieure à 85 et une chrominance inférieure à 2 ; ladite première phase opaque comprenant un agent de conditionnement de la peau ; et
(iii) une seconde phase opaque présentant une tonalité chromatique de 35 à 70, ladite seconde phase opaque comprenant un matériau particulaire opacifiant ;
la phase de base translucide, la première phase et la seconde phase formant un motif stable, visuellement distinct, et la luminosité, la chrominance et la tonalité chromatique étant mesurées selon le procédé décrit dans la description ; et
b) un conteneur permettant de stocker la composition de soins pour la peau à phases multiples, le conteneur étant partiellement translucide.

2. Produit de soins pour la peau selon la revendication 1, dans lequel ledit premier agent actif chronique de soins pour la peau est sélectionné parmi des amino-sucres, des composés B3, des peptides, des composés d'hexamidine et leurs mélanges.

3. Produit de soins pour la peau selon l'une des revendications précédentes, dans lequel la première phase opaque comprend, en outre, 15 % à 30 %, en poids de la première phase opaque, d'un ou plusieurs agents actifs antisolaires.

4. Produit de soins pour la peau selon l'une des revendications précédentes, dans lequel la seconde phase opaque comprend, en outre, un second agent actif chronique de soins pour la peau.

5. Produit de soins pour la peau selon l'une des revendications précédentes, dans lequel la composition de soins pour la peau comprend 55 % à 75 % de la phase de base translucide ; 5 à 15 % de la première phase opaque ; et 20 à 30 % de la seconde phase opaque.

6. Produit de soins pour la peau selon l'une des revendications précédentes, dans lequel la phase de base translucide est un gel aqueux et dans lequel la première phase opaque et la seconde phase opaque sont des émulsions d'huile dans l'eau.

7. Produit de soins pour la peau selon l'une des revendications précédentes, dans lequel la seconde phase opaque présente :
a) une luminosité d'environ 40 à 75 ;
b) une chrominance de 18 à 28 ; et
c) une tonalité chromatique de 45 à 65.

8. Produit de soins pour la peau selon l'une des revendications précédentes, dans lequel la phase de base translucide, la première phase opaque et la seconde phase opaque présentent chacune une viscosité supérieure à 40 000 cps.

9. Produit de soins pour la peau selon l'une des revendications précédentes, dans lequel le conteneur comprend, en outre, un mécanisme de distribution permettant de distribuer la composition de soins pour la peau à phases multiples, la composition de soins pour la peau après distribution présentant un aspect homogène.

10. Produit de soins pour la peau selon la revendication 11, dans lequel la composition de soins pour la peau après distribution présente une tonalité chromatique de 35 à 70.

11. Un ensemble de produits de soins pour la peau comprenant un premier produit de soins pour la peau et un second produit de soins pour la peau, dans lequel :
a) le premier produit de soins pour la peau comprend
(i) une première composition de soins pour la peau non solide, à fonctions multiples et à phases multiples comprenant :
(a) une phase de base translucide
(b) une première phase opaque présentant une luminosité supérieure à 80 et une chrominance inférieure à 5 ; et
(c) une seconde phase opaque présentant une tonalité chromatique de 35 à 70 et une valeur de luminosité ;
la phase de base translucide, la première phase et la seconde phase formant un motif stable, visuellement distinct, et la luminosité, la chrominance et la tonalité chromatique étant mesurées selon le procédé décrit dans la description ; et
(ii) un premier conteneur permettant de stocker la composition de soins pour la peau à phases multiples, le conteneur étant au moins partiellement translucide ; et
b) le second produit de soins pour la peau
(i) une seconde composition de soins pour la peau non solide, à fonctions multiples et à phases multiples comprenant :
(a) une phase de base translucide
(b) une première phase opaque présentant une luminosité supérieure à 80 et une chrominance inférieure à 5 ; et
(c) une seconde phase opaque présentant une tonalité chromatique de 35 à 70 et une valeur de luminosité ;
la phase de base translucide, la première phase et la seconde phase formant un motif stable, visuellement distinct, et la luminosité, la chrominance et la tonalité chromatique étant mesurées selon le procédé décrit dans la description ; et
(ii) un second conteneur permettant de stocker la composition de soins pour la peau à phases multiples, le conteneur étant au moins partiellement translucide ;
la seconde phase opaque de la première composition de soins pour la peau à phases multiples et la seconde phase opaque de la seconde composition de soins pour la peau à phases multiples présentant une différence de luminosité (ΔL) d'au moins 4.
